## Europäisches Patentamt
## European Patent Office
## Office européen des brevets

(19)

(11) Numéro de publication: **0 027 400**
B1

(12)

# FASCICULE DE BREVET EUROPEEN

(45) Date de publication du fascicule du brevet: **13.04.83**

(51) Int. Cl.³: **C 07 C 46/04,** C 07 C 50/12

(21) Numéro de dépôt: **80401371.2** .

(22) Date de dépôt: **26.09.80**

(54) **Perfectionnement à la fabrication de la naphtoquinone-1,4.**

(30) Priorité: **12.10.79 FR 7925426**

(43) Date de publication de la demande:
**22.04.81 Bulletin 81/16**

(45) Mention de la délivrance du brevet:
**13.04.83 Bulletin 83/15**

(84) Etats contractants désignés:
**BE CH DE FR GB IT LI NL**

(56) Documents cités:
**FR - A - 2 168 491**

**INTERNATIONAL JOURNAL OF METHODS IN SYNTHETIC ORGANIC CHEMISTRY, no. 5, 1977 M. PERIASAMY: "A Convenient Method for the Oxidation of Polycyclic Aromatic Hydrocarbons to Quinones" pages 330—332 CHEMICAL ABSTRACTS, vol. 88, no. 3, 16 janvier 1978, ref. 22436r, page 593 Columbus, Ohio, US M. PERIASAMY et al.: "A new 1,2-shift in the oxidation of aromatic rings"**

(73) Titulaire: **P C U K PRODUITS CHIMIQUES UGINE KUHLMANN**
**Service Propriété Industrielle Tour Manhattan**
**F-92087 Paris La Défense 2 Cédex 21 (FR)**

(72) Inventeur: **Jaccaud, Michel**
**38 bis, rue des Granges**
**F-69005 Lyon (FR)**

(74) Mandataire: **Houssin, Jean et al,**
**P C U K Produits Chimiques Ugine Kuhlmann**
**Service Propriété Industrielle Tour Manhattan -**
**Cedex 21**
**F-92087 Paris La Defense 2 (FR)**

Courier Press, Leamington Spa, England.

Perfectionnement à la fabrication de la naphtoquinone-1,4

L'invention a pour objet une amélioration du procédé de fabrication de la naphtoquinone-1,4, plus précisément du procédé qui consiste à oxyder le naphtalène au moyen d'une solution sulfurique de sels cériques.

L'oxydation du naphtalène par les sels cériques est connue depuis de très nombreuses années (USP 729 502 déposé le 26 Mai 1903). Les composés du cérium (IV) se sont révélés les oxydants les plus actifs et les plus sélectifs, eu égard, par exemple, aux composés du vanadium (V), du chrome (VI) et du manganèse (III ou VII). Cette oxydation chimique est les plus souvent, pour des raisons économiques, associée à une régénération électrochimique de l'oxydant. Le brevet français n° 1 591 651 déposé le 23 Octobre 1968, est représentatif de cette technique.

Pour faciliter la séparation des produits, il a été suggéré d'utiliser un solvant non miscible à l'eau: le naphtalène est dissous dans ce solvant et l'oxydant est en solution aqueuse. Les deux phases sont émulsionnées dans le réacteur d'oxydation puis séparées dans un décanteur. La phase aqueuse retourne à la cellule d'électrolyse, alors que la phase organique est traitée pour extraire la naphtoquinone.

La mise en oeuvre d'un tel procédé comporte beaucoup d'inconvénients:

— il n'est pas possible de trouver un solvant totalement inerte: ainsi lorsqu'on utilise une paraffine lourde, il faut opérer en l'absence d'air car il se produit une auto-oxydation du solvant qui génère des sous-produits; ceux-ci s'accumulent sous forme de crasse dans le décanteur et en bloquent le fonctionnement. De plus cette oxydation parasite produit vraisemblablement des peroxydes dangereux.
— il est difficile d'éviter totalement l'entraînement de solvant dans la phase aqueuse; la solubilité même faible de celui-ci peut créer au niveau de la cellule des difficultés technologiques (sensibilité des matériaux plastiques, des diaphragmes aux solvants organiques).
— la productivité du procédé est limitée par la solubilité du produit souvent différente de celle du réactif, C'est le cas du couple naphtalène/naphtoquinone.

Or, il a été trouvé, conformément à la présente invention faite dans les services de la demanderesse, qu'il est possible d'éviter l'utilisation de solvant.

Le procédé selon l'invention a donc pour objet un procédé pour la fabrication de la naphtoquinone-1,4 par oxydation du naphtalène au moyen des sels de cérium (IV), en solution dans un milieu acide aqueux, caractérisé en ce que le naphtalène à l'état pulvérulent est mis en suspension dans la solution du sel cérique au moyen d'un agent dispersant et que la naphtoquinone-1,4- obtenue est séparée du milieu aqueux.

En vue de la réalisation du procédé selon l'invention, on utilise de préférence des agents tensio-actifs anioniques ou non-ioniques. Comme tels on peut citer les alkyl-sulfates ou alkyl-sulfonates, les produites issus de la condensation d'un substrat hydrophile sur un oxyde d'oléfine.

La quantité d'agent dispersant se situe généralement entre 0,01 et 0,5% en poids du naphtalène mis en oeuvre. Pour des raisons économiques, il n'y a pas intérêt à augmenter cette proportion. Le naphtalène pulvérulent utilisé est constitué pratiquement de particules ayant une dimension moyenne inférieure à 25 $\mu$m.

Comme sels de cérium (IV) utilisables dans le procédé selon l'invention, on peut citer le sulfate, le nitrate, l'acétate, le chloro ou le fluoroacétate, etc...

Dans les solutions de chacun de ces sels dans l'acide sulfurique ou l'acide nitrique, on ajoute le dispersant et le naphtalène à l'état de poudre. Le mélange est agité suivant tout moyen connu pour obtenir une suspension homogène.

La transformation en naphtoquinone-1,4 s'effectue, ainsi qu'il est connu dès la température ambiante mais est accélérée sous l'action d'une élévation de la température. La séparation du produit est effectuée alors très simplement par décantation, filtration ou centrifugation et la phase aqueuse est renvoyée à la cellule d'électrolyse pour régénérer l'oxydant. La régénération est conduite suivant les procédés habituels (par exemple: Chemical Engineering, Juin 5, 1967, pages 128—135; brevet américain 3 486 992; Document ORNL—TM—657 R. E. McHenry (Février 1965): "Separation of cerium from other rare earths by electrolytic oxidation and differential extraction"; Przemysl. Chem. 44 (5) 239—43 (1965) Lucja Kohman et Mieczyslaw Pezec: "Electrolytic oxidation of Ce (III) to Ce (IV)"; USP 3 413 203, 18 Août 1965 "Electrolytic oxidation of cerium"; Technol. Rept. Osaka Univ. 10 n° 363—391, 261—9 (1960), Toshio Ishino et Jiro Shiokawa: "Oxidation of cerium by electrolysis".

Le procédé selon l'invention constitue un perfectionnement appréciable à la fabrication de la naphtoquinone-1,4 en ce sens qu'il permet, en particulier, d'obtenir une accélération significative de la vitesse d'attaque du naphtalène; le gain de temps, mis en évidence par les exemples ci-dessous, conduit à une économie importante au stade de l'exploitation en continu du procédé d'oxydation du naphtalène.

## Exemple 1

On introduit 6 g ode naphtalène pulvérulent (diamètre moyen 25 μm) dans 1 200 ml d'une solution sulfurique (100 g/l) de cérium (IV) titrant 0,25 mole de Ce par litre, fortement agitée (1 700 t/mm) à la température de 55°C: la moitié de l'oxydant est consommée en 35 minutes et 86% de l'oxydant sont consommés en 120 minutes.

## Exemple 2

Dans 50 cm³ de solution cérique ayant la composition de celle de l'exemple 1, on introduit 6 g de naphtalène et 30 mg du condensat du nonylphénol (1 mole) sur l'oxyde d'éthylène (10 moles d'OE) et disperse sous ultra-sons. Cette dispersion est mélangée sous agitation dans le réacteur dans les conditions de l'exemple 1: la moitié de l'oxydant est consommée en 10 minutes et 86% de l'oxydant sont consommées en 30 minutes.

## Exemple 3

On introduit 2,4 g de naphtalène micronisé (diamètre moyen 10 μm) dans 450 ml de la solution sulfurique de cérium (IV) à 0,25 mole par litre agitée modérément (580 t/mn) à la température de 55°C: la moitié de l'oxydant est consommée en 45 minutes et 86% de l'oxydant sont consommés en 180 minutes de réaction.

## Exemple 4

On effectue une prédispersion de 2,4 g de naphtalène micronisé (10 μm) dans 10 ml de la solution cérique de l'exemple 1 en présence de dodecyl-benzène-sulfonate de sodium à raison de 0,5% par rapport au naphtalène; cette suspension est introduite dans le réacteur dans les conditions de l'exemple 3: la moitié de l'oxydant est consommée en 10 minutes et 86% de l'oxydant sont consommées en 25 minutes.

## Exemple 5

On introduit 2,4 g de naphtalène broyé (diamètre moyen 25 μm) dans la solution sulfurique de cérium (IV) dans les conditions de l'exemple 3: la moitié de l'oxydant est consommées en 70 minutes et 86% de l'oxydant sont consommées en 200 minutes de réaction.

## Exemple 6

On effectue une prédispersion de 2,4 g de naphtalène broyé (diamètre moyen 25 μm) avec 0,5% de dodecyl-benzène-sulfonate de sodium et introduit cette suspension dans le réacteur, dans les conditions de l'exemple 3. La moitié de l'oxydant est consommée en 12 minutes et 86% de l'oxydant sont consommés en 30 minutes.

## Exemple 7

On reprend les conditions de l'exemple 6 mais avec 0,1% seulement de dispersant: la moitié de l'oxydant est consommée en 16 minutes et 86% de l'oxydant sont consommés en 45 minutes.

## Exemple 8

On reprend les conditions de l'exemple 6 en utilisant comme dispersant le produit résultant de la condensation du nonylphénol (1 mole) avec l'oxyde d'éthylène (10 moles); la moitié de l'oxydant est consommée en 17 minutes et 86% de l'oxydant sont consommées en 45 minutes.

## Revendications

1. Procédé de fabrication de la naphtoquinone-1,4 par oxydation du naphtalène au moyen des sels de cérium (IV) en solution dans un milieu acide aqueux, caractérisé en ce que le naphtalène à l'état pulvérulent est mis en suspension dans la solution du sel cérique au moyen d'un agent dispersant et que la naphtoquinone-1,4 obtenue est séparée du milieu aqueux.

2. Procédé tel que défini sous 1, caractérisé en ce que l'agent dispersant est un dispersant non-ionique.

3. Procédé tel que défini sous 1, caractérisé en ce que l'agent dispersant est un dispersant anionique.

## Patentansprüche

1. Verfahren zur Herstellung von 1,4-Naphthochinon durch Oxidation von Naphthalin mit Cer(IV)-salzen in Lösung in einem saueren wäßrigen Medium, dadurch gekennzeichnet, daß man das Naphthalin in pulverförmigem Zustand mit Hilfe eines Dispergiermittels in der Lösung des Cer(IV)-salzes dispergiert und das erhaltene 1,4-Naphthochinon von dem wäßrigen Medium abtrennt.

2. Verfahren nach Anspruch 1, dadurch gekennzeichnet, daß man als Dispergiermittel ein nichtionisches Dispergiermittel verwendet.

3. Verfahren nach Anspruch 1, dadurch gekennzeichnet, daß man als Dispergiermittel ein anionisches Dispergiermittel verwendet.

## Claims

1. Process for the manufacture of 1,4-naphthoquinone by oxidation of naphthalene by means of cerium (IV) salts in solution in an aqueous acid medium, characterised in that naphthalene, in pulverulent state, is put into suspension in the ceric salt solution by means of a dispersing agent, and that the 1,4-naphthoquinone obtained is separated from the aqueous medium.

2. Process such as defined under 1, characterised in that the dispersing agent is a nonionic dispersant.

3. Process such as defined under 1, characterised in that the dispersing agent is an anionic dispersant.